Europäisches Patentamt

European Patent Office

Office européen des brevets

(19)

(11) Veröffentlichungsnummer: **0 316 690**

**A2**

(12) **EUROPÄISCHE PATENTANMELDUNG**

(21) Anmeldenummer: 88118471.7

(51) Int. Cl.⁴: **A61K 31/55**

(22) Anmeldetag: 05.11.88

(30) Priorität: 17.11.87 DE 3738980

(43) Veröffentlichungstag der Anmeldung:
24.05.89 Patentblatt 89/21

(84) Benannte Vertragsstaaten:
**AT BE CH DE FR GB IT LI LU NL SE**

(71) Anmelder: Dr. Karl Thomae GmbH
Postfach 1755
D-7950 Biberach 1(DE)

(72) Erfinder: Trach, Volker, Dr.
Probststrasse 7
D-7950 Biberach 1(DE)
Erfinder: Eberlein, Wolfgang, Dr. Dipl.-Chem.
Obere Au 6
D-7950 Biberach 1(DE)
Erfinder: Engel, Wolfhard, Dr. Dipl.-Chem.
Mozartstrasse 13
D-7950 Biberach 1(DE)
Erfinder: Mihm, Gerhard, Dr. Dipl.-Chem.
Nickeleshalde 5/1
D-7950 Biberach 1(DE)
Erfinder: Mayer, Norbert, Dr.
Friedrich-Ebert-Strasse 66
D-7950 Biberach 1(DE)
Erfinder: Doods, Henri, Dr.
Hornsteinweg 7
D-7951 Warthausen 1(DE)
Erfinder: Trummlitz, Günter, Dr. Dipl.-Chem.
Buchenweg 27
D-7951 Warthausen 1(DE)

(54) Mittel zur Behandlung von ischämischen Herzerkrankungen.

(57) 6-Chlor-5,10-dihydro-5-[(1-methyl-4-piperidinyl)acetyl]-11H-dibenzo[b,e][1,4]diazepin-11-on und die (+)-Form hiervon, des weiteren die physiologisch verträglichen Säureadditionssalze dieser Verbindungen. eignen sich zur Behandlung von ischämischen Herzerkrankungen, z. B. von Angina pectoris verschiedener Genese.

EP 0 316 690 A2

## Mittel zur Behandlung von ischämischen Herzerkrankungen

Die Erfindung betrifft ein Mittel zur Behandlung von ischämischen Herzerkrankungen, welches die Verbindungen 6-Chlor-5,10-dihydro-5-[(1-methyl-4-piperidinyl)acetyl]-11H-dibenzo[b,e][1,4]diazepin-11-on, ( + )-6-Chlor-5,10-dihydro-5-[(1-methyl-4-piperidinyl)acetyl]-11H-dibenzo[b,e] [1,4]diazepin-11-on und deren physiologisch verträgliche Salze mit anorganischen oder organischen Säuren enthält.

In dem britischen Patent Nr. 2 115 411 (entspricht dem Europäischen Patent Nr. 0 085 892) und dem United States Patent Nr. 4 668 674 werden Verbindungen beschrieben, die als $M_1$-selektive muscarinische Rezeptorantagonisten magensäuresekretionsinhibierende Eigenschaften besitzen und damit vorteilhafterweise zur Behandlung von Magen- und Darmerkrankungen eingesetzt werden können.

Es wurde überraschenderweise gefunden, daß die in den oben angeführten Patenten enthaltenen Verbindungen

I. 6-Chlor-5,10-dihydro-5-[(1-methyl-4-piperidinyl)acetyl]-11H-dibenzo[b,e][1,4]diazepin-11-on

II. ( + )-6-Chlor-5,10-dihydro-5-[(1-methyl-4-piperidinyl)acetyl]-11H-dibenzo[b,e][1,4]diazepin-11-on

und ihre physiologisch verträglichen Salze mit anorganischen oder organischen Säuren noch gänzlich andersgeartete pharmakologische Eigenschaften besitzen, die ihre Anwendung zur Behandlung ischämischer Herzerkrankungen ermöglichen.

In der Literatur sind Fälle beschrieben, wonach therapieresistente Ischämien mit Atropin, einem nicht-selektiven Antimuskarinikum, behandelt wurden.

Ein großer Nachteil der Therapie mit einem nicht-selektiven Antimuskarinikum ist aber die hohe Rate nicht tolerierbarer anticholinerger Nebenwirkungen, wie z. B. von Mydriasis, Salivationshemmung, Obstipation und schwerwiegenden ZNS-Effekten. Diese Nebenwirkungen behindern oder schließen einen Einsatz nicht-selektiver Antimuskarinika in der Dauertherapie aus.

Im Gegensatz zu Atropin wurde für die oben genannten Substanzen I und II überraschenderweise eine ausgeprägte Selektivität für glattmuskuläre coronare Muscarinrezeptoren gefunden. Der große Abstand der erwünschten Effekte an den Coronararterien zu den unerwünschten anticholinergen Wirkungen ermöglicht den Einsatz der Verbindungen I und II bei verschiedenen Formen der coronaren Herzerkrankung, ohne daß mit intolerablen Nebenwirkungen zu rechnen ist.

Diese günstigen Eigenschaften der Verbindungen I und II werden experimentell am Standardmodell der isoliert perfundierten Langendorff-Herzen überprüft. Meßparameter sind Herzfrequenz und Coronarfluß.

Methode:

Männliche Wistar Ratten mit einem Körpergewicht zwischen 300 und 350 g wurden mit Pentobarbital (70 mg/kg i.p.) anaesthe siert. Nach Thorakotomie wurden 500 E Heparin in die Cava inferior injiziert, danach das Herz entnommen und sofort an eine modifizierte Langendorff-Perfusionsapparatur angeschlossen. Die Herzen wurden über die Aorta mit Krebs-Henseleit-Puffer (KHB) retograd bei einem konstanten Druck von 70 mm Hg perfundiert. Die Zusammensetzung des KHB betrug in mM: Kochsalz 114, Kaliumchlorid 4,7, Natriumbicarbonat 23,6, Kaliumdihydrogenphosphat 1,2, Magnesiumsulfat 1,1, Calciumchlorid 2,5. 11 mM Glucose wurden als Substrat beigefügt. Die Herzfrequenz (HR) wurde durch einen Tachographen (IFD, Mülheim) gemessen, der Coronarfluß (CF) wurde mittels eines elektromagnetischen Flowmeters (IFD, Mülheim) in Verbindung mit einer Sonde in dem zuführenden Teil der Aorta bestimmt.

Nach einer 15-minütigen Stabilisierungsphase in allen Experimenten wurde die erste Dosis-Wirkungs-Kurve erstellt. Dies geschah durch die Gabe von dem Fluß angepaßten Bolus-Injektionen (0,01 ml/10 ml CF) mit ansteigenden Dosierungen von Carbachol ($10^{-6}$ bis $10^{-2}$M). Danach wurde die niedrigste Dosis des Antagonisten dem Perfusat beigegeben und die Dosis-Wirkungs-Kurve erneut aufgenommen. Darauffolgend gab man eine höhere Dosis des Antagonisten zu und es wurde der Einfluß von Carbachol auf den Coronarfluß (CF) und die Herzfrequenz (HF) erneut durch die Aufnahme von Dosis-Wirkungs-Kurven quantifiziert.

Auf diese Weise wurden folgende $pA_2$-Werte ($pA_2$-Wert = negativer Logarithmus derjenigen Antagonistenkonzentration, in deren Gegenwart die Agonistenkonzentration verdoppelt werden muß, um wieder 50 % der maximalen Wirkungsstärke zu erreichen) für die Effekte auf den Coronarfluß (CF) sowie die Herzfrequenz (HF) im Vergleich zu Atropin ermittelt:

|  | pA$_2$-Werte | |
| --- | --- | --- |
|  | Coronarflußeffekt<br>CF | Herzfrequenz-Effekt<br>HR |
| Substanz I | 7,9 ± 0,2 | 7,3 ± 0,3 |
| Substanz II | 8,2 ± 0,1 | 7,5 ± 0,2 |
| Atropin | 9,2 ± 0,2 | 9,1 ± 0,2 |

Die Substanzen I und II antagonisierten kompetitiv sowohl den negativen chronotropen Effekt von Carbachol auf das spontanschlagende Herz als auch die Reaktion des Coronarflusses. Anders als Atropin zeigten die Substanzen I und II hochsignifikante Unterschiede hinsichtlich der Beeinflussung von Coronarfluß und Herzfrequenz und zwar wurde die cholinerg induzierte Coronarkonstriktion stärker gehemmt als der Frequenzeffekt des muscarinischen Agonisten.

Die Substanzen I und II besitzen somit eine höhere Affinität für coronare als für frequenzbeeinflussende M-Rezeptoren. Eine derartige selektive Antagonisierung cholinerg bedingter coronarkonstriktorischer Effekte ist für ein Antimuskarinikum völlig überraschend und wurde in der Literatur bisher noch nicht beschrieben.

Zusammenfassend ist zu sagen, daß die Untersuchungsergebnisse für die beiden Verbindungen I und II Coronarselektivität beweisen, die den üblichen Antimuscarinica, wie zum Beispiel Atropin, fehlt. Die unterschiedlichen Affinitäten der Substanzen I und II für muscarinische Rezeptorsubtypen des Myocards eröffnen neue therapeutische Perspektiven für die Behandlung coronarer Gefäßerkrankungen. An therapierbaren Syndromen coronarer Gefäße seien genannt: Alle Formen der stabilen und instabilen Angina pectoris und insbesondere solche vasospastischer Genese.

Die Substanzen können sowohl als Monopräparate als auch in Kombination mit gängigen Coronartherapeutika, wie Calciumantagonisten, β-Blockern und Nitraten eingesetzt werden.

Die beiden Substanzen I und II finden Verwendung zur Herstellung von Arzneimitteln zur Behandlung von ischämischen Herzerkrankungen. Die Substanzen lassen sich hierzu in an sich bekannter Weise in übliche pharmazeutische Zubereitungsformen einarbeiten, z. B. in Lösungen, Ampullen, Suppositorien, Tabletten, Dragées, Kapseln oder Teezubereitungen. Die Tagesdosis liegt im allgemeinen zwischen 0,01 und 0,5, vorzugsweise 0,01 und 0,25 mg/kg Körpergewicht. Die Tagesdosis kann gegebenenfalls auch in Form mehrerer, vorzugsweise 1 bis 3 Einzelgaben, zur Erzielung der gewünschten Wirkung, verabreicht werden.

Die Herstellung der Wirksubstanz I wird in der britischen Patentschrift Nr. 2 115 411, die Gewinnung der (+)-Form dieser Substanz, also der Substanz II, in der US-Patentschrift Nr. 4 668 674 beschrieben.

Die nachfolgenden Beispiele beschreiben die Herstellung einiger pharmazeutischer Zubereitungsformen:

Beispiel I

Tabletten mit 5,0 mg (+)-6-Chlor-5,10-dihydro-5-[(1-methyl-4-piperidinyl)acetyl]-11H-dibenzo[b,e][1,4]-diazepin-11-on

Zusammensetzung:

1 Tablette enthält:

| Wirkstoff | 5,0 mg |
| --- | --- |
| Milchzucker | 152,0 mg |
| Kartoffelstärke | 65,0 mg |
| Magnesiumstearat | 2,0 mg |
| | 224,0 mg |

Herstellungsverfahren:

3

Aus Kartoffelstärke wird durch Erwärmen ein 10%iger Schleim hergestellt. Die Wirksubstanz, Milchzukker und die restliche Kartoffelstärke werden gemischt- und mit obigem Schleim durch ein Sieb der Maschenweite 1,5 mm granuliert. Das Granulat wird bei 45° C getrocknet, nochmals durch obiges Sieb gerieben, mit Magnesiumstearat vermischt und zu Tabletten verpreßt.

| | |
|---|---|
| Tablettengewicht: | 224 mg |
| Stempel: | 9 mm |

Beispiel II

Dragées mit 5,0 mg (+)-6-Chlor-5,10-dihydro-5-[(1-methyl-4-piperidinyl)acetyl]-11H-dibenzo[b,e][1,4]-diazepin-11-on

Die nach Beispiel I hergestellten Tabletten werden nach bekanntem Verfahrem mit einer Hülle überzogen, die im wesentlichen aus Zucker und Talkum besteht. Die fertigen Dragées werden mit Hilfe von Bienenwachs poliert.
Dragéegewicht: 300 mg

Beispiel III

Ampullen mit 1 mg 6-Chlor-5,10-dihydro-5-[(1-methyl-4-piperidinyl) acetyl]-11H-dibenzo[b,e][1,4]diazepin-11-onhydrochlorid

Zusammensetzung:

1 Ampulle enthält:

| | |
|---|---|
| Wirkstoff | 1,0 mg |
| Natriumchlorid | 8,0 mg |
| Dest. Wasser ad | 1 ml |

Herstellungsverfahren:

Die Wirksubstanz und Natriumchlorid werden in dest. Wasser gelöst und anschließend auf das gegebene Volumen aufgefüllt. Die Lösung wird sterilfiltriert und in 1ml-Ampullen abgefüllt.
Sterilisation: 20 Minuten bei 120° C.

Beispiel IV

Suppositorien mit 10 mg 6-Chlor-5,10-dihydro-5-[(1-methyl-4-piperidinyl)acetyl]-11H-dibenzo[b,e][1,4]-diazepin-11-on

Zusammensetzung:

1 Zäpfchen enthält:

| Wirkstoff | 10,0 mg |
|---|---|
| Zäpfchenmasse (z.B. Witepsol W 45 [R]) | 1690,0 mg |
| | 1700,0 mg |

Herstellungsverfahren:

Die feinpulverisierte Wirksubstanz wird in der geschmolzenen und auf 40° C abgekühlten Zäpfchenmasse suspendiert. Man gießt die Masse bei 37° C in leicht vorgekühlte Zäpfchenformen aus. Zäpfchengewicht: 1,7 g

Beispiel V

Tropfen mit (+)-6-Chlor-5,10-dihydro-5-[(1-methyl-4-piperidinyl)acetyl]-11H-dibenzo[b,e][1,4]-diazepin-11-on

Zusammensetzung:

100 ml Tropflösung enthalten:

| p-Hydroxybenzoesäuremethylester | 0,035 g |
|---|---|
| p-Hydroxybenzoesäurepropylester | 0,015 g |
| Anisöl | 0,05 g |
| Menthol | 0,06 g |
| Ethanol rein | 10,0 g |
| Wirkstoff | 0,2 g |
| Natriumcyclamat | 1,0 g |
| Glycerin | 15,0 g |
| Dest. Wasser ad | 100,0 ml |

Herstellungsverfahren:

Die Wirksubstanz und Natriumcyclamat werden in ca. 70 ml Wasser gelöst und Glycerin zugefügt. Man löst die p-Hydroxybenzoesäureester, Anisöl sowie Menthol in Ethanol und fügt diese Lösung unter Rühren der wäßrigen Lösung zu. Abschließend wird mit Wasser auf 100 ml aufgefüllt und schwebeteilchenfrei filtriert.

**Ansprüche**

1.) Verwendung von 6-Chlor-5,10-dihydro-5-[(1-methyl-4-piperidinyl)acetyl]-11H-dibenzo[b,e][1,4]-diazepin-11-on und/oder (+)-6-Chlor-5,10-dihydro-5-[(1-methyl-4-piperidinyl)acetyl]-11H-dibenzo[b,e][1,4]-diazepin-11-on bzw. von physiologisch verträglichen Salzen dieser Verbindungen mit anorganischen oder organischen Säuren zur Behandlung von ischämischen Herzerkrankungen.

2.) Verwendung von 6-Chlor-5,10-dihydro-5-[(1-methyl-4-piperidinyl)acetyl]-11H-dibenzo[b,e][1,4]-diazepin-11-on und/oder (+)-6-Chlor-5,10-dihydro-5-[(1-methyl-4-piperidinyl)acetyl]-11H-dibenzo[b,e][1,4]-diazepin-11-on bzw. von physiologisch verträglichen Salzen dieser Verbindungen mit anorganischen oder organischen Säuren zur Herstellung eines Arzneimittels zur Behandlung von ischämischen Herzerkrankungen.

3.) Verwendung der Verbindungen gemäß Anspruch 1 und 2 zur Bekämpfung von Coronarspasmen.

4.) Verwendung der Verbindungen gemäß Anspruch 1 und 2 zur Behandlung der stabilen und/oder instabilen Angina pectoris.